(19) Europäisches Patentamt
European Patent Office
Office européen des brevets

(11) **EP 3 436 420 B1**

(12) **EUROPÄISCHE PATENTSCHRIFT**

(45) Veröffentlichungstag und Bekanntmachung des Hinweises auf die Patenterteilung:
**04.12.2019 Patentblatt 2019/49**

(21) Anmeldenummer: **17714694.1**

(22) Anmeldetag: **29.03.2017**

(51) Int Cl.:
***C07C 29/149*** (2006.01)

(86) Internationale Anmeldenummer:
**PCT/EP2017/057350**

(87) Internationale Veröffentlichungsnummer:
**WO 2017/167772 (05.10.2017 Gazette 2017/40)**

(54) **VERFAHREN ZUR HYDRIERUNG VON CARBONSÄUREN ZU ALKOHOLEN**

METHOD FOR HYDROGENATING CARBOXYLIC ACIDS IN ORDER TO FORM ALCOHOLS

PROCÉDÉ D'HYDROGÉNATION D'ACIDES CARBOXYLIQUES EN ALCOOLS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.03.2016 EP 16163203**

(43) Veröffentlichungstag der Anmeldung:
**06.02.2019 Patentblatt 2019/06**

(73) Patentinhaber: **BASF SE**
**67056 Ludwigshafen am Rhein (DE)**

(72) Erfinder:
• **PINKOS, Rolf**
**67056 Ludwigshafen (DE)**
• **RITTINGER, Stefan**
**67056 Ludwigshafen (DE)**
• **NUEBLING, Christoph**
**67056 Ludwigshafen (DE)**
• **ABILLARD, Olivier**
**67056 Ludwigshafen (DE)**

(74) Vertreter: **BASF IP Association**
**BASF SE**
**G-FLP-C006**
**67056 Ludwigshafen (DE)**

(56) Entgegenhaltungen:
**US-A1- 2011 124 926**

**Beschreibung**

Die vorliegende Erfindung betrifft ein Verfahren zur kontinuierlichen Hydrierung einer Carbonsäure der allgemeinen Formel (I)

$$Y^1\text{-}X^1\text{-}COOH \qquad\qquad (I)$$

in der $X^1$ für eine $-(CH_2)_n-$ Gruppe mit n gleich 1 bis 10 oder eine $-CH=CH-$Gruppe, und $Y^1$ für H-, HOOC- oder HO-$CH_2$- steht, oder eines Gemisches davon, unter Beibehaltung der Anzahl der Kohlenstoffatome zu einem Alkohol der allgemeinen Formel (II)

$$Y^2\text{-}X^2\text{-}CH_2OH \qquad\qquad (II)$$

in der $X^2$ für eine $-(CH_2)_n-$ Gruppe mit n gleich 1 bis 10, und $Y^2$ für H- oder HO-$CH_2$- steht, mit Wasserstoff bei einer Temperatur von 100 bis 300°C und einem Druck von 10 bis 33 MPa abs in einem axial durchströmten Reaktorrohr mit darin fixiertem Festbettkatalysator, welcher mindestens ein Element aus der Gruppe Re, Co und Cu enthält.

Alkohole stellen eine wichtige Verbindungsklasse mit breitem Verwendungsspektrum dar. So werden diese beispielsweise als Lösungsmittel oder als Zwischenprodukt in der Synthese hochwertiger Verbindungen eingesetzt. Eine mögliche Syntheseroute vieler Alkohole ist dabei die katalytische Oxidation von Kohlenwasserstoffen zu Carbonsäuren und deren anschließende katalytische Hydrierung. 1,4-Butandiol wird somit technisch durch Oxidation von n-Butan zu Maleinsäureanhydrid, dessen Hydrolyse zu Maleinsäure und/oder dessen Veresterung zu Dialkylmaleat, und deren nachfolgende Hydrierung gewonnen, wobei dabei intermediär auch Bernsteinsäure und 4-Hydroxybuttersäure gebildet werden. 1,6-Hexandiol wird technisch durch Hydrierung von Adipinsäure sowie 6-Hydroxycapronsäure hergestellt, wobei beide Säuren als Nebenprodukt bei der großtechnischen Oxidation von Cyclohexan zu Cyclohexanon und Cyclohexanol anfallen. Zudem wird Adipinsäure auch als Reinstoff durch Salpetersäureoxidation von Cyclohexanol gewonnen. 1,4-Butandiol und 1,6-Hexandiol sind wichtige Zwischenprodukte in der Herstellung von Polymeren.

US 5,698,749 beschreibt die Herstellung von 1,4-Butandiol durch Hydrierung von Maleinsäure, Maleinsäureanhydrid, Fumarsäure, Bernsteinsäure, Bernsteinsäureanhydrid, Bernsteinsäuredimethylester oder gamma-Butyrolacton als Ausgangsstoff an einem Katalysator, welcher mindestens ein Element aus der Gruppe Re, W und Mo umfasst. Die Hydrierung erfolgt dabei diskontinuierlich in einem Autoklaven oder kontinuierlich in einem Festbettreaktor im geraden Durchgang unter Einsatz einer relativ hoch konzentrierten wässrigen Lösung mit einem Gehalt an Ausgangsstoff von bis zu 50 Gew.-%.

Nachteilig an dieser Fahrweise ist der Restgehalt an nicht umgesetzter Carbonsäure, welcher sowohl zu einer verringerten Ausbeute an Wertprodukt als auch aufgrund der Restsäure zu Nachteilen in der Produktqualität führt.

US 3,478,112 beschreibt die Hydrierung von Carbonsäuren zu den entsprechenden Alkoholen an einem Katalysator enthaltend Co sowie mindestens ein Element aus der Gruppe Cu, Cr und Mn. Die zu hydrierende Carbonsäure kann auch als wässrige Lösung eingesetzt werden. Weiterhin lehrt das US-Patent den Einsatz einer durch Produktalkohol verdünnten CarbonsäureLösung. Die Hydrierung erfolgt dabei diskontinuierlich in einem Autoklaven oder kontinuierlich in einem Festbettreaktor im geraden Durchgang.

Trotz der in US 3,478,112 gelehrten Verdünnung der einzusetzenden Carbonsäure ist der Carbonsäuregehalt der Einsatzlösung mit den aus den Beispielen abgeschätzten Bereich von etwa 25 bis 50 Gew.-% Carbonsäure relativ hoch. Entsprechend treffen die in der Würdigung von US 5,698,749 genannten Nachteile einer verringerten Ausbeute an Wertprodukt sowie einer verminderten Produktqualität durch die vorhandene Restsäure auch für das Verfahren nach US 3,478,112 zu.

US 2011/0,124,926 lehrt die Herstellung von Diolen durch Hydrierung entsprechender Carbonsäuren, Carbonsäureanhydride oder Lactone an einem Co-haltigen Festbettkatalysator unter Zuführung von Alkali- oder Erdalkali-Ionen. Die zu hydrierende Carbonsäure kann in Reinsubstanz oder als Lösung wie beispielsweise in Wasser oder einem Alkohol eingesetzt werden. Trotz entsprechender Verdünnung ist der Säuregehalt der Carbonsäurelösung relativ hoch und liegt beispielsweise in Beispiel 1 bei über 36 Gew.-%. Um die bei der Hydrierung gebildete Wärme zu entfernen, lehrt die US-Schrift die partielle Rückführung des Hydrieraustrags.

Nachteilig an dieser Fahrweise ist der relativ hohe Gehalt an nicht-umgesetzter Carbonsäure im Reaktoraustrag, was zu einer verringerten Ausbeute an Wertprodukt sowie zu einer verminderten Produktqualität durch die vorhandene Restsäure führt. So offenbart selbst Beispiel 2 bei der Hydrierung eines Adipinsäure und 6-Hydroxycapronsäure enthaltenden Gemisches unter Zusatz von NaOH einen Reaktoraustrag, welcher neben 27 bis 28 Gew.-% 1,6-Hexandiol noch 1,4 bis 2,3 Gew.-% 6-Hydroxycapronsäure enthält.

DE 2,321,101 offenbart die Hydrierung von Carbonsäuren zu den entsprechenden Alkoholen an einem Katalysator enthaltend Co, Cu und Mn sowie Mo. Die Hydrierung erfolgt an einem Katalysatorfestbett oder in Suspension in wässriger und/oder alkoholischer Lösung. Die DE-Offenlegung lehrt dabei auch den Einsatz von Produktalkohol als Verdünnungsmittel.

Trotz der in DE 2,321,101 gelehrten Verdünnung der einzusetzenden Carbonsäure und der in Beispiel 1a erwähnten partiellen Rückführung des hydrierten Gemischs, ist der Carbonsäuregehalt des Reaktorzulaufs relativ hoch. So weist das Zulaufgemisch in Beispiel 1a eine Säurezahl von 62 mg KOH/g und der Reaktoraustrag eine Säurezahl von 1,8 mg KOH/g auf. Somit befinden sich im Reaktoraustrag noch deutliche Mengen an nicht hydrierter Restsäure. Entsprechend treffen die in der Würdigung von US 5,698,749 genannten Nachteile einer verringerten Ausbeute an Wertprodukt sowie einer verminderten Produktqualität durch die vorhandene Restsäure auch für das Verfahren nach DE 2,321,101 zu.

US 4,940,805 offenbart die Herstellung von 1,4-Butandiol, Tetrahydrofuran und gamma-Butyrolacton durch Hydrierung von Maleinsäure oder Bernsteinsäure sowie deren Anhydride und Ester an einem Katalysator enthaltend Co sowie mindestens ein Element aus der Gruppe Cu, P und Mo. Der zu hydrierende Ausgangsstoff kann dabei als Schmelze vorliegen oder in Alkohol gelöst sein und zudem Wasser enthalten. Als bevorzugte Fahrweise ist die kontinuierliche Hydrierung an einem Katalysatorfestbett in Riesel- oder Sumpffahrweise mit partieller Rückführung des Hydrieraustrags genannt.

So beschreibt Beispiel 1 in US 4,940,805 die Hydrierung einer wässrigen Lösung mit 40 Gew.-% Maleinsäure an einem $H_3PO_4$, CoO, CuO, $Mn_3O_4$ und $MoO_3$ enthaltenden Festbettkatalysator in Rieselfahrweise mit einem Frischzulauf der genannten Lösung von 200 g/h und einer Rückführrate von 9 l/h. Unter diesen Bedingungen wurde eine Ausbeute an 1,4-Butandiol von nur 29,6 bis 38,8%, bezogen auf die eingesetzte Maleinsäure, erreicht.

Nachteilig an dem in US 4,940,805 beschriebenen Verfahren ist die Bildung eines Gemisches mit erheblichen Mengen an Tetrahydrofuran und gamma-Butyrolacton und somit die damit verbundene geringe Ausbeute an 1,4-Butandiol.

Aufgabe der vorliegenden Erfindung war es, ein Verfahren zur kontinuierlichen Hydrierung gesättigter oder ungesättigter Carbonsäuren zu den entsprechenden Alkoholen zu finden, welches die oben genannten Nachteile nicht oder nur stark vermindert aufweist, und insbesondere eine nahezu vollständige Hydrierung der Carbonsäuren zu den entsprechenden Alkoholen bei gleichzeitiger Vermeidung einer nennenswerten Überhydrierung der Alkohole ermöglicht. Entsprechend soll das neue Verfahren einen hohen Carbonsäure-Umsatz bei hoher Selektivität zu den entsprechenden Alkoholen aufweisen. Zudem soll das Verfahren technisch einfach durchführbar sein und sich für die Nachhydrierung eines, noch Carbonsäuren enthaltenden Stroms aus der technischen Herstellung von Diolen, wie beispielsweise 1,4-Butandiol oder 1,6-Hexandiol, welcher durch Vorhydrierung höher konzentrierter Carbonsäure-Lösungen gewonnen wurde, eignen.

Überraschend wurde ein Verfahren zur kontinuierlichen Hydrierung einer Carbonsäure der allgemeinen Formel (I)

$$Y^1\text{-}X^1\text{-COOH} \qquad (I)$$

gefunden, in der $X^1$ für eine $-(CH_2)_n-$ Gruppe mit n gleich 1 bis 10 oder eine -CH=CH-Gruppe, und $Y^1$ für H-, HOOC- oder $HO\text{-}CH_2-$ steht, oder eines Gemisches davon, unter Beibehaltung der Anzahl der Kohlenstoffatome zu einem Alkohol der allgemeinen Formel (II)

$$Y^2\text{-}X^2\text{-}CH_2OH \qquad (II)$$

in der $X^2$ für eine $-(CH_2)_n-$ Gruppe mit n gleich 1 bis 10, und $Y^2$ für H- oder $HO\text{-}CH_2-$ steht, mit Wasserstoff bei einer Temperatur von 100 bis 300°C und einem Druck von 10 bis 33 MPa abs in einem axial durchströmten Reaktorrohr mit darin fixiertem Festbettkatalysator, welcher mindestens ein Element aus der Gruppe Re, Co und Cu enthält, und welches dadurch gekennzeichnet ist, dass die zu hydrierende Carbonsäure (I) in einem flüssigen Gemisch (III) enthaltend die Carbonsäure (I), Wasser und Alkohol (II) vorliegt, wobei das Gemisch (III)

a) eine Säurezahl von 0,2 bis 25 mg KOH/g aufweist,
b) mindestens 15 Gew.-% Wasser,
c) mindestens 20 Gew.-% Alkohol (II) enthält, und
d) die, auf die geometrische Querschnittsfläche des leeren, katalysatorfreien Reaktorrohres berechnete Strömungsgeschwindigkeit der durchströmenden Flüssigkeit 10 bis 50 m/h beträgt.

Ausgangsstoff für das erfindungsgemäße Verfahren ist ein Gemisch (III) enthaltend die zu hydrierende Carbonsäure (I), Wasser und Alkohol (II).

Bei der Carbonsäure (I) handelt es sich dabei um eine Carbonsäure der allgemeinen Formel (I)

$$Y^1\text{-}X^1\text{-COOH} \qquad (I)$$

in der $X^1$ für eine $-(CH_2)_n-$ Gruppe mit n gleich 1 bis 10 oder eine -CH=CH-Gruppe, und $Y^1$ für H-, HOOC- oder HO-$CH_2$- steht. Konkret umfasst die Gruppe der zu hydrierenden Carbonsäure (I)

$CH_3$-COOH (Essigsäure),

$CH_3$-$CH_2$-COOH (Propionsäure),

$CH_3$-$(CH_2)_2$-COOH (Buttersäure),

$CH_3$-$(CH_2)_3$-COOH (Valeriansäure),

$CH_3$-$(CH_2)_4$-COOH (Capronsäure),

$CH_3$-$(CH_2)_5$-COOH (Önanthsäure),

$CH_3$-$(CH_2)_6$-COOH (Caprylsäure),

$CH_3$-$(CH_2)_7$-COOH (Pelargonsäure),

$CH_3$-$(CH_2)_8$-COOH (Caprinsäure),

$CH_3$-$(CH_2)_9$-COOH (Undecansäure),

$CH_2$=CH-COOH (Acrylsäure),

HOOC-$CH_2$-COOH (Malonsäure),

HOOC-$(CH_2)_2$-COOH (Bernsteinsäure),

HOOC-$(CH_2)_3$-COOH (Glutarsäure),

HOOC-$(CH_2)_4$-COOH (Adipinsäure),

HOOC-$(CH_2)_5$-COOH (Pimelinsäure),

HOOC-$(CH_2)_6$-COOH (Korksäure),

HOOC-$(CH_2)_7$-COOH (Azelainsäure),

HOOC-$(CH_2)_8$-COOH (Sebacinsäure),

HOOC-$(CH_2)_9$-COOH (Undecandisäure)

HOOC-$(CH_2)_{10}$-COOH (Dodecandisäure),

cis HOOC-CH=CH-COOH (Maleinsäure),

trans HOOC-CH=CH-COOH (Fumarsäure),

HO-$CH_2$-$CH_2$-COOH (3-Hydroxypropionsäure),

HO-$CH_2$-$(CH_2)_2$-COOH (4-Hydroxybuttersäure),

HO-$CH_2$-$(CH_2)_3$-COOH (5-Hydroxyvaleriansäure),

HO-$CH_2$-$(CH_2)_4$-COOH (6-Hydroxycapronsäure),

HO-CH$_2$-(CH$_2$)$_5$-COOH (7- Hydroxyönanthsäure),

HO-CH$_2$-(CH$_2$)$_6$-COOH (8-Hydroxycaprylsäure),

HO-CH$_2$-(CH$_2$)$_7$-COOH (9-Hydroxypelargonsäure),

HO-CH$_2$-(CH$_2$)$_8$-COOH (10-Hydroxycaprinsäure),

HO-CH$_2$CH$_2$)$_9$-COOH (11-Hydroxyundecansäure),

HO-CH$_2$-(CH$_2$)$_{10}$-COOH (12-Hydroxydodecansäure),

cis HO-CH$_2$-CH=CH-COOH (4-Hydroxyisocrotonsäure)

sowie

trans HO-CH$_2$-CH=CH-COOH (4-Hydroxycrotonsäure).

Natürlich kommen auch Gemische der genannten Carbonsäuren infrage.

Bevorzugt steht X$^1$ für eine -(CH$_2$)$_n$- Gruppe mit n gleich 1 bis 4 oder eine -CH=CH- Gruppe. Von den hierbei bevorzugten Carbonsäuren (I) sind vor allem Essigsäure, Bernsteinsäure, 4-Hydroxybuttersäure, Maleinsäure, Fumarsäure, Glutarsäure, 5-Hydroxyvaleriansäure, Adipinsäure, 6-Hydroxycapronsäure sowie deren Gemische bevorzugt.

Besonders bevorzugt steht X$^1$ für eine -(CH$_2$)$_n$- Gruppe mit n gleich 2 bis 4 oder eine -CH=CH-Gruppe. Unabhängig davon steht Y$^1$ besonders bevorzugt für eine HOOC- oder HO-CH$_2$- Gruppe. Unter Berücksichtigung der besonders bevorzugten Gruppen für X$^1$ und Y$^1$, handelt es sich bei der zu hydrierenden Carbonsäure (I) um Bernsteinsäure, 4-Hydroxybuttersäure, Maleinsäure, Fumarsäure, Glutarsäure, 5-Hydroxyvaleriansäure, Adipinsäure, 6-Hydroxycapronsäure sowie deren Gemische.

Ganz besonders bevorzugt sind Bernsteinsäure, 4-Hydroxybuttersäure, Maleinsäure, Glutarsäure, 5-Hydroxyvaleriansäure, Adipinsäure, 6-Hydroxycapronsäure sowie deren Gemische, insbesondere Bernsteinsäure, 4-Hydroxybuttersäure, Maleinsäure, Adipinsäure, 6-Hydroxycapronsäure sowie deren Gemische.

Die genannte Carbonsäure (I) wird dabei unter Beibehaltung der Anzahl der Kohlenstoffatome zu einem Alkohol der allgemeinen Formel (II)

Y$^2$-X$^2$-CH$_2$OH (II)

in der X$^2$ für eine -(CH$_2$)$_n$- Gruppe mit n gleich 1 bis 10, und Y$^2$ für H- oder HO-CH$_2$- steht, katalytisch hydriert. Aus den -COOH Gruppen bildet sich durch die Hydrierung jeweils eine -CH$_2$OH Gruppe. Enthält die Carbonsäure (I) zudem eine -CH=CH- Gruppe, so bildet sich daraus eine -CH$_2$-CH$_2$- Gruppe.

Entsprechend der eingesetzten Carbonsäure (I) steht X$^2$ des daraus gebildeten Alkohols (II) bevorzugt für eine -(CH$_2$)$_n$- Gruppe mit n gleich 1 bis 4. Von den hierdurch bevorzugten Alkoholen sind vor allem Ethanol, 1,4-Butandiol, 1,5-Pentandiol, 1,6-Hexandiol sowie deren Gemische bevorzugt.

Besonders bevorzugt steht X$^2$ für eine -(CH$_2$)$_n$- Gruppe mit n gleich 2 bis 4. Unabhängig davon steht Y$^2$ besonders bevorzugt für eine HO-CH$_2$- Gruppe. Unter Berücksichtigung der besonders bevorzugten Gruppen für X$^2$ und Y$^2$, handelt es sich bei den Alkoholen (II) um 1,4-Butandiol, 1,5-Pentandiol, 1,6-Hexandiol sowie deren Gemische, insbesondere um 1,4-Butandiol, 1,6-Hexandiol sowie deren Gemische.

So werden beispielsweise Bernsteinsäure, 4-Hydroxybuttersäure, Maleinsäure und Fumarsäure zu 1,4-Butandiol, Glutarsäure und 5-Hydroxyvaleriansäure zu 1,5-Pentandiol und Adipinsäure und 6-Hydroxycapronsäure zu 1,6-Hexandiol hydriert.

Wie bereits erwähnt, ist eine nahezu vollständige Hydrierung der Carbonsäuren zu den entsprechenden Alkoholen bei gleichzeitiger Vermeidung einer nennenswerten Überhydrierung der Alkohole wünschenswert. Überraschend wurde dabei gefunden, dass dieses Ziel erreicht wird, wenn man ein flüssiges Gemisch (III) enthaltend die Carbonsäure (I), Wasser und Alkohol (II) einsetzt, wobei das Gemisch (III)

a) eine Säurezahl von 0,2 bis 25 mg KOH/g aufweist,
b) mindestens 15 Gew.-% Wasser, und
c) mindestens 20 Gew.-% Alkohol (II) enthält,
die Hydrierung bei einer Temperatur von 100 bis 300°C und einem Druck von 10 bis 33 MPa abs in einem axial durchströmten Reaktorrohr mit darin fixiertem Festbettkatalysator, welcher mindestens ein Element aus der Gruppe Re, Co und Cu enthält, durchgeführt wird und

d) die, auf die geometrische Querschnittsfläche des leeren, katalysatorfreien Reaktorrohres berechnete Strömungsgeschwindigkeit der durchströmenden Flüssigkeit 10 bis 50 m/h beträgt.

Der Gehalt der zu hydrierenden Carbonsäure (I) ist durch die Säurezahl des Gemisches (III) definiert. Die Säurezahl ist eine chemische Größe zur Charakterisierung von sauren Bestandteilen in Gemischen. Sie bezeichnet die Masse an Kaliumhydroxid in mg, die notwendig ist, um die in 1 g des Gemisches enthaltenen Säuren zu neutralisieren. Die Säurezahl wird bestimmt wird durch

(i) vermischen von 1,5 g des zu untersuchenden Gemisches bei einer Temperatur von 20°C mit 10 mL Pyridin, 50 mL Tetrahydrofuran, 5 mL Wasser und 10 Tropfen einer Lösung enthaltend 0,1 Gew.-% Phenolphthalein in Ethanol,
(ii) titrieren der Mischung mit einer Lösung enthaltend 0,1 Mol.-% Kaliumhydroxid in Ethanol,
(iii) ermitteln der bis Erreichen des Umschlagspunktes von farblos zu rosa hinzugegebenen Menge an Kaliumhydroxid, und
(iv) umrechnen der ermittelten Menge an Kaliumhydroxid auf die Einheit mg KOH pro g Gemisch.

Die Säurezahl des zu hydrierenden Gemischs (III) beträgt beim erfindungsgemäßen Verfahren 0,2 bis 25 mg KOH/g. Die diesem Bereich entsprechende Menge an Carbonsäure (I) ist abhängig von der Molmasse der Carbonsäure sowie der Anzahl der Säuregruppen. So entspricht dieser Bereich im Falle eines Gemischs enthaltend Wasser, Alkohol und die einbasige Carbonsäure 6-Hydroxycapronsäure (Molmasse 132,16 g/Mol) einem Gehalt von 0,047 bis 5,89 Gew.-% 6-Hydroxycapronsäure. Im Falle eines entsprechenden Gemischs mit der zweibasigen Bernsteinsäure entspricht dieser Bereich einem Gehalt von 0,021 bis 2,63 Gew.-% Bernsteinsäure.

Die Säurezahl des zu hydrierenden Gemischs (III) beträgt bevorzugt ≥ 0,5 mg KOH/g und besonders bevorzugt ≥ 1 mg KOH/g, sowie bevorzugt ≤ 20 mg KOH/g, besonders bevorzugt ≤ 15 mg KOH/g und ganz besonders bevorzugt ≤ 10 mg KOH/g.

Der Gehalt an Wasser im einzusetzenden Gemisch (III) beträgt mindestens 15 Gew.-% und höchstens 80 Gew.-% abzüglich der im Gemisch (III) enthaltenen Menge an Carbonsäure (I). Der Gehalt an Wasser beträgt bevorzugt mindestens 20 Gew.-%, besonders bevorzugt mindestens 30 Gew.-% und ganz besonders bevorzugt mindestens 50 Gew.-%.

Der Gehalt an Alkohol (II) im einzusetzenden Gemisch (III) beträgt mindestens 20 Gew.-% und höchstens 85 Gew.-% abzüglich der im Gemisch (III) enthaltenen Menge an Carbonsäure (I). Der Gehalt an Alkohol (II) beträgt bevorzugt mindestens 25 Gew.-%, besonders bevorzugt mindestens 30 Gew.-% und ganz besonders bevorzugt mindestens 45 Gew.-%.

Die Summe der Gehalte an Wasser und Alkohol (II) im einzusetzenden Gemisch (III) beträgt bevorzugt mindestens 60 Gew.-% und besonders bevorzugt mindestens 85 Gew.-%.

Neben der zu hydrierenden Carbonsäure (I), Wasser und Alkohol (I) kann das einzusetzende Gemisch (III) noch weitere Komponenten in unterschiedlichen Mengen enthalten sein. Somit beträgt die Summe der Gehalte an Carbonsäure (I), Wasser und Alkohol (II) im Gemisch (III) ≤ 100 Gew.-%. Enthält das Gemisch (III) keine weiteren Komponenten, so beträgt die genannte Summe 100 Gew.-%. Enthält das Gemisch (III) weiteren Komponenten, so beträgt die genannte Summe < 100 Gew.-%. Bevorzugt beträgt die Summe der Gehalte an Carbonsäure (I), Wasser und Alkohol (II) im Gemisch (III) 40 bis 100 Gew.-%.

Das beim erfindungsgemäßen Verfahren einzusetzende Gemisch (III) kann unterschiedlichen Ursprungs sein. So ist es beispielsweise möglich, das Gemisch (III) durch Mischen der einzelnen Komponenten herzustellen. Es ist aber auch möglich und im Allgemeinen sogar vorteilhaft, das Gemisch (III) durch eine katalytische Vorhydrierung einer Lösung, welche die Carbonsäure (I) und Wasser enthält, zu gewinnen. Bei dieser wird zwar die Carbonsäure (I) in erster Linie zum Alkohol (II) hydriert, es bleibt jedoch auch eine gewisse Restmenge an Carbonsäure (I) übrig, so dass das erhaltene Gemisch Carbonsäure (I), Wasser und Alkohol (II) enthält.

Als Reaktoren werden beim erfindungsgemäßen Verfahren Apparate eingesetzt, welche ein oder mehrere parallele Reaktorrohre umfassen. Die Reaktorrohre sind gekennzeichnet durch eine Querschnittsfläche CSA und eine Länge L. Im Allgemeinen beträgt der Quotient aus der Länge und der Quadratwurzel der Querschnittsfläche

$$1 \leq \frac{L}{\sqrt{CSA}} \leq 1000 .$$

Bevorzugt beträgt L/√CSA ≥ 2, besonders bevorzugt ≥ 5 und ganz besonders bevorzugt ≥ 10, sowie bevorzugt ≤ 750, besonders bevorzugt ≤ 500 und ganz besonders bevorzugt ≤ 100. Sind mehrere Reaktorrohre hintereinandergeschaltet, so werden zur Berechnung des genannten Quotienten die Längen addiert und der Mittelwert der bezüglich der Längen gewichteten Querschnittsfläche verwendet. Die absolute Länge, beziehungsweise bei hintereinandergeschalteten Reaktorrohren deren Gesamtlänge, beträgt üblicherweise 0,5 m bis 50 m, bevorzugt ≥ 1 m, besonders bevorzugt ≥ 4 m, sowie bevorzugt ≤ 8 m und besonders bevorzugt ≤ 10 m.

Als Beispiele geeigneter Reaktoren seien Schachtreaktoren und Rohrbündelreaktoren genannt. Rohrbündelreaktoren sind durch den Einsatz mehrerer paralleler Reaktorrohre gekennzeichnet. Auch wenn definitionsgemäß bereits zwei parallel verschaltete Reaktorrohre einen Rohrbündelreaktor bilden, liegt die übliche Anzahl der parallelen Rohre in einem Rohrbündelreaktor bei 50 bis 22000.

Üblicherweise sind die Reaktorrohre vertikal ausgerichtet. Sie werden axial durchströmt und können sowohl in Sumpffahrweise, das heißt von unten nach oben, als auch in Rieselfahrweise, also von oben nach unten, mit dem flüssigen Gemisch (III) durchströmt werden. Die Zufuhr des gasförmigen Wasserstoffs kann sowohl im Gleichstrom als auch im Gegenstrom erfolgen. Bevorzugt erfolgt die Zufuhr von Wasserstoff im Gleichstrom.

Als Hydrierkatalysator wird beim erfindungsgemäßen Verfahren ein sogenannter Festbettkatalysator eingesetzt, welcher im Reaktorrohr fixiert vorliegt. Die Fixierung des Festbettkatalysators erfolgt üblicherweise durch dem Fachmann bekannte Methoden. So kann dieser beispielsweise auf einem Trägerboden oder auf einer Schüttung inerter Kugeln, etwa Steatitkugeln, aufliegen.

Der beim erfindungsgemäßen Verfahren einzusetzende Festbettkatalysator enthält mindestens ein Element aus der Gruppe Rhenium (Re), Cobalt (Co) und Kupfer (Cu). Bevorzugt sind Katalysatoren, welche ein Element aus der Gruppe Re und Co enthalten. Grundsätzlich können die Katalysatoren neben den genannten Elementen noch weitere metallische und nicht-metallische Elemente enthalten.

Der einzusetzende Festbettkatalysator kann unter Verwendung des üblichen Fachwissens auf verschiedene Art und Weise hergestellt werden. So kann dieser beispielsweise durch Tränkung oder Imprägnierung eines Trägers, durch Fällung auf einen Träger, durch Fällung des gesamten Materials oder durch andere, dem Fachmann bekannte Methoden hergestellt werden.

Beim Einsatz eines Re enthaltenden Festbettkatalysator enthält dieser im Allgemeinen 0,1 bis 10 Gew.-% Re, bezogen auf den reduzierten Festbettkatalysator. Neben Re kann dieser gegebenenfalls noch weitere Metalle als katalytisch wirksame Komponenten enthalten. Als Beispiele seien Pd, Ru, Pt, Sn, Co, Cu und Fe genannt. Werden weitere Metalle als katalytisch wirksame Komponenten eingesetzt, so liegt deren Gehalt üblicherweise im Bereich von Spuren bis zu 15 Gew.-%, bezogen auf den reduzierten Festbettkatalysator.

Bei den Re enthaltenden Festbettkatalysatoren handelt es sich bevorzugt um Trägerkatalysatoren. Diese werden bevorzugt durch Tränkung oder Imprägnierung geeigneter Träger mit geeigneten Re-Salzen oder durch Auffällung von Re ausgeeigneten Re-Salzen auf einen geeigneten Träger hergestellt. Geeignete und zugleich auch bevorzugte Träger sind Kohlen, wie zum Beispiel Graphit oder Aktivkohle, $ZrO_2$, $Al_2O_3$, $SiO_2$, $TiO_2$ oder Gemische davon, sowie Verbindungen daraus mit Zeolithen oder Tone. Besonders bevorzugt als Träger ist Aktivkohle. Die Träger können sowohl als bereits vorgefertigte Formkörper, als auch beispielsweise pulverförmig zur Tränkung, Imprägnierung oder Auffällung eingesetzt werden. Als zur Tränkung, Imprägnierung oder Auffällung geeignete Re-Salze seien beispielsweise $NH_4ReO_4$ oder $Re_2O_7$ genannt, welche beispielsweise in Form einer wässrigen Lösung vorliegen. Bei der Auffällung werden die Re-Salze, welche beispielsweise als wässrige Lösung vorliegen, mitreduzierende Stoffe wie Wasserstoff oder Hydrazin reduziert und somit Re aufgefällt. Die erhaltene Katalysatorvorstufe wird anschließend üblicherweise getrocknet. Bevorzugt erfolgt die Trocknung bei 80 bis 150°C, besonders bevorzugt bei 100 bis 130°C. Wurde ein pulverförmiger Träger eingesetzt, so erfolgt nach der Trocknung noch die Formgebung, beispielsweise durch Tablettierung. Entsprechende Methoden zur Herstellung von Re enthaltenden Trägerkatalysatoren, einschließlich der weiteren Verarbeitung zum einsatzfähigen Festbettkatalysator, sind dem Fachmann bekannt. Die Herstellung von Re enthaltenden Festbettkatalysatoren ist beispielsweise in US 2003/0,114,719 und der darin zitierten Literatur beschrieben.

Bei den Co beziehungsweise Cu enthaltenden Festbettkatalysatoren handelt es sich bevorzugt um Fällkatalysatoren. Sie können aber auch durch Tränkung, Imprägnierung oder anderen, dem Fachmann bekannte Methoden hergestellt werden.

Im Falle eine Co enthaltenden Festbettkatalysators enthält dieser im Allgemeinen 1 bis 90, bevorzugt 10 bis 85 und besonders bevorzugt 25 bis 80 Gew.-% Co, bezogen auf den reduzierten Festbettkatalysator. Zur Fällung setzt man eine Lösung eines geeigneten Co-Salzes ein. Geeignete Co-Salze sind beispielsweise $Co(NO_3)_2$, Co-Acetat oder Co-Chlorid. Üblicherweise werden diese in Wasser gelöst und bilden eine saure Lösung mit einem pH-Wert von < 7. Zur Fällung wird die Lösung in der Regel in einem durchmischten Behälter, beispielsweise einem Rührkessel, vorgelegt und eine geeignete basische Lösung als Fällungsagenz zur Erhöhung des pH-Wertes zugeführt. Als geeignete basische Lösung sei beispielsweise eine wässrige $Na_2CO_3$-Lösung (Sodalösung) genannt. Dadurch steigt der pH-Wert und das gebildete, basische Co-Salz fällt aus. Im Falle einer Sodalösung als Fällungsagenz bildet sich beispielsweise Co-Carbonat.

Im Falle eine Cu enthaltenden Festbettkatalysators enthält dieser im Allgemeinen 0,5 bis 60, bevorzugt 2 bis 55 und besonders bevorzugt 5 bis 50 Gew.-% Cu, bezogen auf den reduzierten Festbettkatalysator. Die Fällung eines Cu enthaltenden Festbettkatalysators erfolgt ähnlich der eines Co enthaltenden Festbettkatalysators. Geeignete Cu-Salze sind beispielsweise $Cu(NO_3)_2$, Cu-Acetat oder Cu-Chlorid. Üblicherweise werden diese in Wasser gelöst und bilden eine saure Lösung mit einem pH-Wert von < 7. Zur Fällung wird die Lösung in der Regel in einem durchmischten Behälter, beispielsweise einem Rührkessel, vorgelegt und eine geeignete basische Lösung als Fällungsagenz zur Erhöhung des pH-Wertes zugeführt. Als geeignete basische Lösung sei beispielsweise eine wässrige $Na_2CO_3$-Lösung (Sodalösung) genannt. Dadurch steigt der pH-Wert und das gebildete, basische Cu-Salz fällt aus. Im Falle einer Sodalösung als Fällungsagenz bildet sich beispielsweise Cu-Carbonat.

Es ist auch möglich und gegebenenfalls vorteilhaft, Co-Salz und Cu-Salz analog der obigen Beschreibung gemeinsam zu fällen, um einen Co und Cu enthaltenden Festbettkatalysator herzustellen. Zudem ist gegebenenfalls auch möglich, weitere Metallsalze aus geeigneten Lösungen zusammen mit Co und/oder Cu-Salzen auszufällen. Als weitere Metalle seien beispielsweise Mo, Ti, Zr, Sn oder Mn genannt. Im Falle von Mo, Ti, Zr, Sn und Mn stellen auch hier wässrige Lösungen der Nitrate geeignete Ausgangsstoffe dar.

Um die Fällung zu vervollständigen, ist es in der Regel von Vorteil, die frisch gefällte Suspension unter weiterer Durchmischung für einige Zeit, beispielsweise für 10 Minuten bis 24 Stunden, bevorzugt 1 bis 5 Stunden zu belassen und bei einem Absinken des pH-Wertes auf einen Wert unterhalb pH 6 basisches Fällungsagenz nachzudosieren.

Die erhaltene Suspension wird anschließend abfiltriert, gewaschen, beispielsweise mit Wasser, und getrocknet. Bevorzugt erfolgt die Trocknung bei 80 bis 150°C, besonders bevorzugt bei 100 bis 130°C. Um die Oxide zu erhalten wird der getrocknete Filterkuchen anschließend im Luftstrom kalziniert. Bevorzugt erfolgt die Kalzinierung bei 250 bis 700°C, besonders bevorzugt bei 300 bis 600°C und ganz besonders bevorzugt bei 400 bis 600°C. Die Dauer der Kalzinierung beträgt üblicherweise 0,1 bis 10 Stunden, bevorzugt 0,3 bis 5 Stunden.

Zur Formgebung wird das kalzinierte Oxid mit Wasser oder einer wässrigen Lösung angeteigt und geknetet. Als zum Anteigen geeignete wässrigen Lösungen seien beispielsweise ammoniakalische Lösungen von Promotor-Metallen genannt. Die geknetete Masse wird anschließend in einer Strangpresse zu Strängen extrudiert. Die erhaltenen Stränge werden getrocknet und nachfolgend kalziniert. Bevorzugt erfolgt die Trocknung bei 80 bis 150°C, besonders bevorzugt bei 100 bis 130°C. Die Kalzinierung erfolgt bevorzugt bei 300 bis 700°C und besonders bevorzugt bei 400 bis 600°C.

Alternativ zur Formgebung über Verstrangung ist es auch möglich, das kalzinierte Oxid zu tablettieren. Hierzu wird es im Allgemeinen mit Tablettierhilfsmitteln, wie etwa Graphitpulver oder Cu-Pulver versetzt und zu Tabletten geformt.

Des Weiteren kann der Festbettkatalysator auch Alkali- und Erdalkalimetalle, zumeist in ionischer Form, enthalten. Explizit genannt seien hier Na, K, Mg und Ca. Der Gehalt der Alkali- und Erdalkalimetalle beträgt in Summe im Allgemeinen 0 bis 5 Gew.-% und bevorzugt 0 bis 2 Gew.-%, bezogen auf den reduzierten Festbettkatalysator. Besonders vorteilhaft ist die Gegenwart von Alkali- und Erdalkalimetall bei Co beziehungsweise Cu enthaltenden Festbettkatalysatoren. Bei Co beziehungsweise Cu enthaltenden Fällungskatalysatoren erfolgt die Zugabe der Alkali- und Erdalkalimetalle üblicherweise durch Zugabe einer Alkali- beziehungsweise Erdalkalimetalle enthaltenden Lösung während der Fällung. Idealerweise werden die Alkali- beziehungsweise Erdalkalimetalle über das Fällungsagenz eingebracht. So führt beispielsweise der Einsatz einer Sodalösung dazu, dass der Fällkatalysator anschließend Na enthält.

In einer bevorzugten Ausführungsform des erfindungsgemäßen Verfahrens setzt man einen Festbettkatalysator ein, welcher 30 bis 85 Gew.-% Co und 0,1 bis 2 Gew.-% Na und/oder K, bezogen auf den reduzierten Festbettkatalysator, enthält.

Die Herstellung von Co beziehungsweise Cu enthaltenden Festbettkatalysatoren ist beispielsweise in DE-A 23 21 101 beschrieben.

Beim erfindungsgemäßen Verfahren setzt man bevorzugt

- einen Trägerkatalysator enthaltend 0,1 bis 10 Gew.-% Re auf einen Träger aus der Gruppe enthaltend Graphit, Aktivkohle, $ZrO_2$, $Al_2O_3$, $SiO_2$ und $TiO_2$,
- einen Fällkatalysator enthaltend 1 bis 90 Gew.-% Co,
- einen Fällkatalysator enthaltend 0,5 bis 60 Gew.-% Cu, oder
- einen Fällkatalysator enthaltend 15 bis 85 Gew.-% Co und 5 bis 20 Gew.-% Cu, wobei die Summe aus dem Gehalt an Co und Cu 100 Gew.-% nicht übersteigt,

jeweils bezogen auf den reduzierten Festbettkatalysator ein.

Der beim erfindungsgemäßen Verfahren einzusetzende Re, Co und/oder Cu enthaltende Festbettkatalysator besteht im Allgemeinen aus Formkörpern. Üblicherweise setzt man Formkörper des gleichen Typs und der gleichen Größe ein. Es können jedoch auch Mischungen verschiedener Formkörpertypen und Formkörpergrößen eingesetzt werden. Als Formkörpertypen eignen sich insbesondere Tabletten, Kugeln und Stränge, wobei die Querschnittsform von der eines ausgefüllten Kreises auch abweichen kann. So sind beispielsweise sternförmige Tabletten oder Stränge, sowie durchgehende Hohlräume wie etwa bei Lochtabletten, möglich. Bevorzugt sind Tabletten sowie strangförmige Formkörper. Die Abmessungen der Formkörper liegen im Allgemeinen im Bereich von 2 mm bis 5 cm. Bei Tabletten, Kugeln und Strängen liegt der bevorzugte Durchmesser bei 2 bis 10 mm. Im Falle von Tabletten beträgt deren Höhe bevorzugt 2 bis 6 mm. Im Falle von Strängen beträgt deren Länge bevorzugt 3 bis 20 mm.

Bevorzugt wird der Re, Co und/oder Cu enthaltende Festbettkatalysator vor seinem Einsatz in der katalytischen Hydrierung der Carbonsäure (I) mit Wasserstoff aktiviert. Die Aktivierung kann innerhalb oder außerhalb des Hydrierreaktors durchgeführt werden. Wird diese außerhalb des Hydrierreaktors durchgeführt, so wird der Festbettkatalysator hierzu üblicherweise in ein Rohr eingebaut, welches die Durchleitung von Wasserstoff ermöglicht. Im Falle eines Re enthaltenden Festbettkatalysators erfolgt die Aktivierung vorteilhafterweise bei einer Temperatur von 200 bis 350°C und einem Druck von 0,1 bis 1 MPa abs durch Hindurchleiten von Wasserstoff über einen Zeitraum von 0,5 bis 24 Stunden. Im Falle eines Co und/oder Cu enthaltenden Festbettkatalysators erfolgt die Aktivierung vorteilhafterweise bei einer Temperatur von 130 bis 330°C und einem Druck von 0,1 bis 1 MPa abs durch Hindurchleiten von Wasserstoff über einen Zeitraum von 3 bis 72 Stunden. Wurde der Festbettkatalysator außerhalb des Hydrierreaktors aktiviert, so ist dieser vor der Überführung in den Hydrierreaktor durch vorsichtiges Hindurchleiten eines oxidierend wirkenden Gases, bevorzugt eines Stickstoff und Sauerstoff enthaltenden Stroms, zu passivieren. Verfahren zur Aktivierung und Passivierung von Re beziehungsweise Co und/oder Cu enthaltenden Festbettkatalysatoren sind dem Fachmann allgemein bekannt.

Der beim erfindungsgemäßen Verfahren einzusetzende Wasserstoff kann sowohl unverdünnt als auch mit Inertgas, beispielsweise Stickstoff, verdünnt zugeführt werden. Vorteilhaft ist die Zufuhr eines Wasserstoff enthaltenden Gases mit einem möglichst hohen Gehalt an Wasserstoff. Bevorzugt ist ein Gehalt an Wasserstoff von ≥ 80 Vol.-%, besonders bevorzugt von ≥ 90 Vol.-% und ganz besonders bevorzugt von ≥ 95 Vol.-%.

Die Menge an einzusetzendem Wasserstoff sollte so bemessen sein, dass am Reaktorausgang mindestens ein kleiner Teil unverbraucht vorliegt. Bevorzugt ist die zugeführte Menge an Wasserstoff so zu bemessen, dass ≥ 5%, besonders bevorzugt ≥ 10% und ganz besonders bevorzugt ≥ 15% des zugeführten Wasserstoffs unverbraucht am Reaktorausgang vorliegen. Bevorzugt liegen am Reaktorausgang ≤ 90% und besonders bevorzugt ≤ 80% der zugeführten Menge an Wasserstoff vor.

Das erfindungsgemäße Verfahren führt man bei einer Temperatur von 100 bis 300°C, bevorzugt bei ≥ 130°C und besonders bevorzugt bei ≥ 150°C, sowie bevorzugt bei ≤ 250°C und besonders bevorzugt bei ≤ 240°C durch. Der Druck beträgt dabei 10 bis 33 MPa abs, bevorzugt ≥ 15 MPa abs und besonders bevorzugt ≥ 20 MPa abs, sowie bevorzugt ≤ 30 MPa abs und besonders bevorzugt ≤ 28 MPa abs.

Ein wesentliches Merkmal der Erfindung ist die Bereitstellung einer, auf die geometrische Querschnittsfläche des leeren, katalysatorfreien Reaktorrohres berechnete Strömungsgeschwindigkeit der durchströmenden Flüssigkeit von 10 bis 50 m/h. Erst durch diese überraschende Maßnahme wird eine nahezu vollständige Hydrierung der Carbonsäuren zu den entsprechenden Alkoholen bei gleichzeitiger Vermeidung einer nennenswerten Überhydrierung der Alkohole erreicht.

Die, auf die geometrische Querschnittsfläche des leeren, katalysatorfreien Reaktorrohres berechnete Strömungsgeschwindigkeit der durchströmenden Flüssigkeit ist der berechnete Quotient $v$ (in m/h) aus dem Volumenstrom der durchströmenden Flüssigkeit $\dot{V}$ (in m³/h) und der geometrischen Querschnittsfläche des leeren, katalysatorfreien Reaktorrohres CSA (in m²)

$$v = \frac{\dot{V}}{CSA} \,.$$

Die geometrische Querschnittsfläche des leeren, katalysatorfreien Reaktorrohres CSA als Rechengröße ist unabhängig von Menge und Geometrie des eingesetzten Festbettkatalysators. So besitzt etwa ein Reaktorrohr mit dem inneren Durchmesser ID die geometrische Querschnittsfläche

$$CSA = \left(\frac{ID}{2}\right)^{2} \pi \,.$$

Steigt die, auf die geometrische Querschnittsfläche des leeren, katalysatorfreien Reaktorrohres berechnete Strömungsgeschwindigkeit der durchströmenden Flüssigkeit auf Werte über 50 m/h, steigt der Restgehalt an Carbonsäuren im Hydrieraustrag. Eine nahezu vollständige Hydrierung der Carbonsäuren zu den entsprechenden Alkoholen wird somit nicht mehr erreicht. Der gewünschte Alkohol ist somit weiterhin mit den unerwünschten Carbonsäuren verunreinigt. Sinkt die, auf die geometrische Querschnittsfläche des leeren, katalysatorfreien Reaktorrohres berechnete Strömungsgeschwindigkeit der durchströmenden Flüssigkeit auf Werte unterhalb 10 m/h, sinkt auch der Gehalt des gewünschten Alkohols infolge Überhydrierung oder Etherbildung. Folge ist letztendlich der Verlust an Wertprodukt und eine Verringerung der Ausbeute.

Die, auf die geometrische Querschnittsfläche des leeren, katalysatorfreien Reaktorrohres berechnete Strömungsgeschwindigkeit der durchströmenden Flüssigkeit beträgt bevorzugt ≥ 15 m/h und besonders bevorzugt ≥ 20 m/h, sowie bevorzugt ≤ 45 m/h und besonders bevorzugt ≤ 40 m/h.

Die Katalysatorbelastung liegt beim erfindungsgemäßen Verfahren in der Regel im Bereich von 0,1 bis 50 m$^3$ flüssiges Gemisch (III) pro m$^3$ Schüttvolumen des eingesetzten Festbettkatalysators und Stunde, oder abgekürzt 0,1 bis 50 m$^3$/m$^3$h. Bevorzugt beträgt die Katalysatorbelastung ≥ 0,5 m$^3$/m$^3$h und besonders bevorzugt ≥ 1 m$^3$/m$^3$h, sowie bevorzugt ≤ 20 m$^3$/m$^3$h und besonders bevorzugt ≤ 10 m$^3$/m$^3$h.

Da aufgrund der relativ niedrigen Säurezahl des flüssigen Gemischs (III) von 0,2 bis 25 mg KOH/g die Wärmeentwicklung bei der Hydrierung ebenfalls relativ niedrig ist, ist eine Kühlung des Reaktors nicht erforderlich, wenngleich natürlich auch gekühlte Reaktoren eingesetzt werden können. Üblicherweise führt man die erfindungsgemäße Hydrierung adiabat durch. Die adiabate Temperaturerhöhung liegt üblicherweise im Bereich von 1 bis 30°C, bevorzugt im Bereich von 1,5 bis 20°C und besonders bevorzugt im Bereich von 2 bis 10°C.

Das erfindungsgemäße Verfahren kann sowohl mit als auch ohne Rückführung von hydriertem Gemisch durchgeführt werden. Wird das Verfahren mit Rückführung durchgeführt, so führt man im Allgemeinen dem zu hydrierenden Gemisch (III) bis zu 75 Gew.-% und gegebenenfalls bis zu 50 Gew.-% des hydrierten Gemischs zu. Neben einer Erhöhung des Volumenstroms der durch den Reaktor hindurchzuleitenden Flüssigkeit wird durch die Rückführung auch die Konzentration der zu hydrierenden Carbonsäure (I) abgesenkt. Entsprechend sinkt somit im Falle einer adiabaten Fahrweise auch die adiabate Temperaturerhöhung. Da diese aufgrund der relativ niedrigen Säurezahl des flüssigen Gemischs (III) auch ohne Rückführung bereits relativ niedrig ist, kann im Allgemeinen auf eine Rückführung verzichtet werden.

Bevorzugt wird daher das erfindungsgemäße Verfahren ohne Rückführung von hydriertem Gemisch durchgeführt.

Wie bei der Beschreibung des Festbettkatalysators bereits erwähnt, kann dieser auch Alkali- und Erdalkalimetalle enthalten. Alkali- und Erdalkalimetalle haben einen selektivitätssteigerden Effekt. Sie fördern die gewünschte Hydrierung der Carbonsäure (I) zu den Alkoholen (II) und verringern die unerwünschte Überhydrierung der Alkohole. Allerdings verliert der Festbettkatalysator während der Hydrierung Alkali- beziehungsweise Erdalkalimetallionen durch sogenanntes Ausbluten. Um diesem Effekt entgegenzuwirken und während des Betriebs eine hohe Selektivität sicherzustellen, ist es vorteilhaft, Alkali- beziehungsweise Erdalkalimetalle in Form von, im Gemisch (III) löslicher Salze während des Betriebs zuzuführen. Da Natrium und Kalium besonders leicht verfügbar sind, ist die Zufuhr dieser Metalle in Form ihrer entsprechenden Salze bevorzugt. Allerdings sollte der pKs-Wert der korrespondierenden Säure dieser Salze kleiner als der pKs-Wert von Ameisensäure sein. Besonders bevorzugt führt man die besonders gut verfügbaren Hydroxide oder Carboxylate von Na oder K zu.

Das zu hydrierende Gemisch (III) enthält bevorzugt 10 bis 1000 Gew.-ppm Alkali- oder Erdalkalimetalle, besonders bevorzugt ≥ 20 Gew.-ppm, insbesondere ≥ 50 Gew.-ppm, sowie besonders bevorzugt ≤ 500 Gew.-ppm, insbesondere ≤ 400 Gew.-ppm.

In einer besonders bevorzugten Ausführungsform enthält das Gemisch (III) 10 bis 1000 Gew.-ppm Alkalimetall aus der Gruppe Na und K.

Durch die erfindungsgemäße Hydrierung wird ein hydriertes Gemisch mit einer, im Vergleich zum eingesetzten Gemisch (III) deutlich niedrigeren Säurezahl erhalten. Auch beim Einsatz eines Gemischs (III) mit einer ursprünglichen Säurezahl von 25 mg KOH/g ist somit ein hydriertes Gemisch mit einer Säurezahl von < 0,5 mg KOH/g möglich.

Aus dem hydrierten Gemisch können nachfolgend beispielsweise die gewünschten Alkohole (II) isoliert werden. Die Isolierung kann im Allgemeinen gemäß dem üblichen Fachkönnen beispielsweise destillativ erfolgen. So können die gewünschten Alkohole (II) in hoher Reinheit gewonnen werden. Reinheiten von ≥ 99 Gew.-% und bevorzugt ≥ 99,5 Gew.-% sind somit möglich.

Wie bereits weiter oben erwähnt, ist es möglich und im Allgemeinen sogar notwendig, das, beim erfindungsgemäßen Verfahren zu hydrierende Gemisch (III) durch eine katalytische Vorhydrierung einer Lösung, welche die Carbonsäure (I) und Wasser enthält, zu gewinnen. Derartige Lösungen entstehen beispielsweise als Zwischenprodukt in der gezielten Herstellung der Alkohole (II). So kann beispielsweise 1,4-Butandiol durch Hydrierung von Maleinsäure gewonnen werden, welche wiederum nach bekannten Methoden durch katalytische Oxidation von n-Butan zu Maleinsäureanhydrid und anschließende Hydrolyse erhalten wird. 1,5-Pentandiol kann etwa durch Hydrierung von Glutarsäure oder 5-Hydroxyvaleriansäure gewonnen werden, welche wiederum durch Oxidation von Cyclopentanon erhältlich sind. 1,6-Hexandiol kann beispielsweise durch Hydrierung von Adipinsäure oder 6-Hydroxycapronsäure gewonnen werden, welche als Nebenprodukte in der katalytische Oxidation von Cyclohexan zu Cyclohexanon und Cyclohexanol anfallen. Derartige, durch Oxidation gewonnene Lösungen enthalten die entsprechenden Carbonsäuren (I) in teilweise hohen Konzentrationen. Die Säurezahl derartiger Lösungen liegt somit üblicherweise im Bereich von 50 bis 900 mg KOH/g, bevorzugt im Bereich von 100 bis 900 mg KOH/g.

Die genannten Lösungen können nach dem Fachmann bekannten Verfahren hydriert werden. Entsprechende Verfahren werden beispielsweise kontinuierlich in Gegenwart eines heterogenen Hydrierkatalysators durchgeführt. Geeignete Hydrierkatalysatoren enthalten beispielsweise ein oder mehrere Elemente aus der 7 bis 11 Gruppe des Periodensystems, bevorzugt Re, Ru, Co, Pd, Pt, Cu. Geeignete Verfahren sind beispielsweise in US 2011/0,124,926 oder US 5,698,749 vorbeschrieben. Bei diesen Hydrierungen wird zwar die Carbonsäure (I) in erster Linie zum Alkohol (II) hydriert, es bleibt jedoch auch eine gewisse Restmenge an Carbonsäure (I) übrig, so dass das erhaltene Gemisch üblicherweise Carbonsäure (I), Wasser und Alkohol (II) enthält und eine Zusammensetzung wie die des Gemischs (III) aufweist.

In einer bevorzugten Ausführungsform des erfindungsgemäßen Verfahrens gewinnt man daher das zu hydrierende Gemisch (III) durch kontinuierliche Vorhydrierung einer Lösung enthaltend Carbonsäure (I) und Wasser, wobei die Lösung eine Säurezahl von 50 bis 900 mg KOH/g aufweist.

Entsprechend der relativ hohen Säurezahl ist auch die bei der Vorhydrierung freigesetzte Wärme relativ hoch. Daher ist es vorteilhaft, die Vorhydrierung zur besseren Wärmeabfuhr mit Rückführung der vorhydrierten Lösung durchzuführen. Vorteilhafterweise führt man 50 bis 98 Gew.-% der vorhydrierten Lösung zur Vorhydrierung zurück.

In einer bevorzugten Ausführungsform zur Hydrierung von Bernsteinsäure zu 1,4-Butandiol wird ein Bernsteinsäure, Wasser und 1,4-Butandiol enthaltendes Gemisch (III), welches eine Säurezahl von 0,2 bis 10 mg KOH/g aufweist, mindestens 15 Gew.-% Wasser und mindestens 20 Gew.-% 1,4-Butandiol enthält, bei einer Temperatur von 130 bis 250°C und einem Druck von 15 bis 28 MPa abs in Gegenwart eines Re, Co und/oder Cu enthaltenden Festbettkatalysators in einem axial durchströmten Reaktorrohr mit einer, auf die geometrische Querschnittsfläche des leeren, katalysatorfreien Reaktorrohres berechneten Strömungsgeschwindigkeit der durchströmenden Flüssigkeit von 10 bis 50 m/h, und in Anwesenheit von 10 bis 1000 Gew.-ppm Na-Ionen im geraden Durchgang ohne Rückführung hydriert. Das in der erfindungsgemäßen Hydrierung einzusetzende Gemisch (III) wird dabei bevorzugt durch Vorhydrierung einer Maleinsäure enthaltenden Lösung mit einer Säurezahl im Bereich von 100 bis 900 mg KOH/g an einem Re, Ru, Co, Pd, Pt und/oder Cu enthaltenden Festbettkatalysator erzeugt, wobei die Vorhydrierung mit einer Rückführung von 50 bis 98 Gew.-% der vorhydrierten Lösung erfolgt. Das durch die erfindungsgemäße Hydrierung erhaltene Gemisch enthält nur noch Spuren an Bernsteinsäure und weist eine Säurezahl von < 0,5 mg KOH/g auf.

In einer bevorzugten Ausführungsform zur Hydrierung von 6-Hydroxycapronsäure und Adipinsäure zu 1,6-Hexandiol wird ein 6-Hydroxycapronsäure, Adipinsäure, Wasser und 1,6-Hexandiol enthaltendes Gemisch (III), welches eine Säurezahl von 0,2 bis 10 mg KOH/g aufweist, mindestens 15 Gew.-% Wasser und mindestens 20 Gew.-% 1,6-Hexandiol enthält, bei einer Temperatur von 130 bis 250°C und einem Druck von 15 bis 28 MPa abs in Gegenwart eines Re, Co und/oder Cu enthaltenden Festbettkatalysators in einem axial durchströmten Reaktorrohr mit einer, auf die geometrische Querschnittsfläche des leeren, katalysatorfreien Reaktorrohres berechneten Strömungsgeschwindigkeit der durchströmenden Flüssigkeit von 10 bis 50 m/h, und in Anwesenheit von 10 bis 1000 Gew.-ppm Na-Ionen im geraden Durchgang ohne Rückführung hydriert. Das in der erfindungsgemäßen Hydrierung einzusetzende Gemisch (III) wird dabei bevorzugt durch Vorhydrierung einer 6-Hydroxycapronsäure und Adipinsäure enthaltenden Lösung mit einer Säurezahl im Bereich von 100 bis 900 mg KOH/g an einem Re, Ru, Co, Pd, Pt und/oder Cu enthaltenden Festbettkatalysator erzeugt, wobei die Vorhydrierung mit einer Rückführung von 50 bis 98 Gew.-% der vorhydrierten Lösung erfolgt. Das durch die erfindungsgemäße Hydrierung erhaltene Gemisch enthält nur noch Spuren an 6-Hydroxycapronsäure und Adipinsäure und weist eine Säurezahl von < 0,5 mg KOH/g auf.

Das erfindungsgemäße Verfahren ermöglicht die kontinuierliche Hydrierung gesättigter und ungesättigter Carbonsäuren zu den entsprechenden Alkoholen bei nahezu vollständiger Hydrierung der Carbonsäuren zu den entsprechenden Alkoholen und gleichzeitiger Vermeidung einer nennenswerten Überhydrierung der Alkohole. Es ermöglicht einen hohen Carbonsäure-Umsatz bei hoher Selektivität zu den entsprechenden Alkoholen. Es ist zudem technisch einfach durchführbar. Das Verfahren eignet sich insbesondere zur Nachhydrierung eines, noch Carbonsäuren enthaltenden Stroms aus der technischen Herstellung von Diolen, wie beispielsweise 1,4-Butandiol oder 1,6-Hexandiol, welcher durch Vorhydrierung höher konzentrierter Carbonsäure-Lösungen gewonnen wurde.

Beispiele

Beispiele 1 bis 4 (Hydrierung von Bernsteinsäure zu 1,4-Butandiol)

Herstellung eines Re/Pd-Katalysators

(5% Re, 5% Pd auf oxidierter Aktivkohle)

Jeweils 500 g Aktivkohle der Korngröße 30 x 70 Mesh wurden mit einem Überschuss (überstehende Lösung) an konzentrierter Salpetersäure (69-71 %ige $HNO_3$) versetzt und bei 80°C für etwa 18 Stunden gerührt. Nach dem Abkühlen wurde das Produkt durch Filtration isoliert, mehrmals mit einem Überschuss an Wasser gewaschen und bei 120°C getrocknet.

Jeweils 1,5 kg der durch die oben genannten Ansätze hergestellten oxidierten Aktivkohle wurden dann mit 7,2 kg einer wässrigen Lösung enthaltend 114 g $NH_4ReO_4$ und 1,09 kg einer wässrigen $Pd(NO_3)_2$-Lösung mit einem Pd-Gehalt von 7,26 Gew.-% vermischt und die erhaltene Aufschlämmung zur Trockene eingedampft, sowie anschließend bei 120°C getrocknet und zu 3 x 3 mm Tabletten tablettiert.

Herstellung eines vorhydrierten Einsatzstoffes durch Hydrierung von Maleinsäure

3,00 Liter der, nach der oben genannten Methode hergestellten Re/Pd-Katalysatortabletten wurden nun in ein 10 m langes Reaktorrohr mit 2 cm Innendurchmesser gefüllt und darin zunächst aktiviert. Hierzu wurde der Katalysator in einem Wasserstoffstrom mit 1°C pro Minute auf 200°C aufgeheizt und dann bei 200°C für 5 Stunden im Wasserstoffstrom gehalten.

An diesem Katalysator wurde nun ein Strom von 0,5 kg/h einer 33 Gew.%-Lösung von Maleinsäure in Wasser bei 20 MPA abs, 150 bis 170°C und einer Produktrückführrate von 5 kg/h unter Zufuhr von 250 NL/h Wasserstoff in kontinuierlicher Fahrweise hydriert und der Reaktionsaustrag gesammelt. Nach 4 Wochen kontinuierlicher Fahrweise wurde das Verfahren gestoppt und der gesammelte Reaktionsaustrag analysiert.

Der so vorhydrierte Einsatzstoff hatte einen Wassergehalt von ca. 75 Gew.-%. Als organische Komponenten enthielt er gemäß gaschromatografischer Analyse, wasserfrei gerechnet, ca. 90,7 Gew.-% 1,4-Butandiol, ca. 2,4 Gew.-% Tetrahydrofuran, ca. 2,5 Gew.-% n-Butanol, ca. 0,5 Gew.-% gamma-Butyrolacton sowie weitere Reaktionsprodukte der Maleinsäure. Die Säurezahl des vorhydrierten Einsatzstoffs betrug 3,8 mg KOH/g und war überwiegend auf Bernsteinsäure zurückzuführen.

Hydrierung von Bernsteinsäure im vorhydrierten Einsatzstoff zu 1,4-Butandiol

Zur Hydrierung der Bernsteinsäure wurden 3,00 Liter der, nach der oben genannten Methode hergestellten Re/Pd-Katalysatortabletten in ein 10 m langes Reaktorrohr mit 2 cm Innendurchmesser gefüllt und zunächst aktiviert. Hierzu wurde der Katalysator in einem Wasserstoffstrom mit 1°C pro Minute auf 200°C aufgeheizt und dann bei 200°C für 5 Stunden im Wasserstoffstrom gehalten.

Anschließend wurde der vorhydrierte Einsatzstoff kontinuierlich bei 170°C Reaktoreintrittstemperatur, 20 MPa abs und 70 NL/h Wasserstoff pro kg vorhydriertem Einsatzstoff in Rieselfahrweise über den Katalysator geleitet. Bei der vorliegenden Versuchsreihe wurde die Zulaufmenge an vorhydriertem Einsatzstoff sukzessive gesteigert, wobei die Probennahme jeweils ca 24 Stunden nach Einstellung einer Zulaufmenge erfolgte.

Die Ergebnisse sind in Tabelle 1 dargestellt. Der Wassergehalt wurde nach Karl-Fischer und der Gehalt an 1,4-Butandiol gaschromatografisch ermittelt.

Die Beispiele 1 bis 4 zeigen die Abhängigkeit der Säurezahl sowie des Gehaltes an Zielalkohol von der Strömungsgeschwindigkeit der Flüssigkeit im Rohrreaktor.

Bei einer geringen Strömungsgeschwindigkeit von nur 5 m/h (Beispiel 1) wird die vorhandene Carbonsäure zwar weitgehend vollständig hydriert, was sich in einer sehr geringen Säurezahl von < 0,5 mg KOH/g zeigt, jedoch werden dabei auch nennenswerte Mengen des Zielalkohols 1,4-Butandiol durch Hydrierung abgebaut. So sinkt der Gehalt von 1,4-Butandiol von ursprünglich 90,7 Gew.-% auf 88,0 Gew.-%, jeweils wasserfrei gerechnet.

Eine hohe Strömungsgeschwindigkeit von 60 m/h (Beispiel 4) ergibt zwar eine hohe rechnerische Durchsatzzahl durch den Reaktor, ermöglicht aber nur noch eine teilweise Hydrierung der vorhandenen Carbonsäure. So weist der Austrag noch eine Säurezahl von 1,5 mg KOH/g auf, was einer Hydrierung von nur etwa 60% der vorhandenen Carbonsäure entspricht.

Demgegenüber zeigen die Beispiele 2 und 3 mit einer Strömungsgeschwindigkeit von 10 beziehungsweise 30 m/h sowohl durch eine Säurezahl im Austrag von < 0,5 mg KOH/g eine nahezu vollständige Hydrierung der vorhandenen Carbonsäure, als auch durch einen deutlich gestiegenen Gehalt an 1,4-Butandiol von 94,5 beziehungsweise 94,3 Gew.-% eine deutliche Zunahme an Zielalkohol 1,4-Butandiol.

Durch die erfindungsgemäße Hydrierung wurde aus einem Alkohol und Carbonsäure enthaltenden Einsatzstrom ein nahezu carbonsäurefreier Produktstrom mit einem deutlichen Zuwachs an Zielalkohol erhalten.

Beispiele 5 bis 8 (Hydrierung von 6-Hydroxycapronsäure zu 1,6-Hexandiol)

Herstellung eines Co/Cu/Mn/Mo-Katalysators

(66% CoO, 20% CuO, 7,3% $Mn_3O_4$, 3,6% $MoO_3$, 0,15 % $Na_2O$, 3% $H_3PO_4$)

Der Co/Cu/Mn-Katalysatorprecursor wurde durch zweistufiges Fällen eines Ausgangsgemisches aus 38,3 kg einer 12,6 Gew.-% Kobalt enthaltenden, wässrigen Kobaltnitratlösung, 6,53 kg einer 15,3 Gew.-% Kupfer enthaltenden, wässrigen Kupfernitratlösung, 2,78 kg einer 12,6 Gew.-% Mangan enthaltenden, wässrigen Mangannitratlösung und 0,199 kg 75,3 Gew.-%iger Phosphorsäure mit 20 Gew.-%iger Sodalösung hergestellt. Dabei wurde in einem ersten Rührbehälter (Nutzvolumen 6 L) bei 50°C Ausgangsgemisch kontinuierlich in einer Menge zugeführt, welche 1,5 kg Metalloxid/h entspricht, und unter Rühren mit so viel Sodalösung versetzt, um einen pH-Wert von 8,5 (gemessen mit einer Glaselektrode) aufrechtzuhalten. Die unvollständige Fällung wird komplett in einen zweiten Behälter überführt und dann bei einem pH von 6,5 bis 7,5 (gegebenenfalls unter Zugabe weiterer Sodalösung) innerhalb von 2 Stunden nachgefällt. Die erhaltene Suspension wurde abfiltriert, gewaschen und getrocknet.

Hierduch wurde ein basisches Carbonat mit einer BET-Oberfläche von etwa 120 $m^2/g$ erhalten. Dieses Carbonat wurde nun bei einer Temperatur im Bereich von 420 bis 540°C im Luftstrom zu Oxid zersetzt und mit vollentsalztem Wasser restliches Alkali herausgewaschen. 4 kg des gewaschenen und getrockneten Oxids wurden nun in einem Kneter mit 652 g einer ammoniakalischer Mo-Lösung, welche durch Auflösen von technischem Molybdänoxidhydrat in wäßriger Ammoniaklösung hergestellt wurde und einen rechnerischen $MoO_3$-Gehalt von 25,5 Gew.-% aufwies, versetzt und durch Kneten vermischt. Während des Knetens wurde der durch die Waschprozesse ausgetragene Phosphor durch frische Phosphorsäure ersetzt, sowie 285 g einer 65,3 Gew.-%igen Salpetersäure und 1300 g vollentsalztes Wasser eingebracht und 2,5 Stunden intensiv geknetet. Die geknetete Masse wurde dann zu Strangpreßlingen mit einem Durchmesser von 4 mm und einer Länge von 3 bis 9 mm verformt, getrocknet und 6 Stunden bei 500°C kalziniert. Die Strangpreßlinge wiesen ein Schüttgewicht von 1700 g/L auf.

Hydrierung von 6-Hydroxycapronsäure zu 1,6-Hexandiol

3,00 Liter des beschriebenen Co/Cu/Mn/Mo-Katalysators wurden nun in ein 10 m langes Reaktorrohr mit 2 cm Innendurchmesser gefüllt. Der zu hydrierende Einsatzstoff wurde durch Vorhydrierung eines 17 Gew.-% Adipinsäure, 16 Gew.-% 6-Hydroxycapronsäure, 2 Gew.-% Glutarsäure, 1,5 Gew.-% 5-Hydroxypentansäure, 1 Gew.-% Ameisensäure, 1 Gew.-% 1,4-Cyclohexandiol, 1 Gew.-% 1,2-Cyclohexandiol und 0,3 Gew.-% Cyclohexanol/Cyclohexanon enthaltenden Gemisches, welches als Nebenprodukt bei der Oxidation von Cyclohexan zu Cyclohexanol/Cyclohexanon anfällt und durch Wasserwäsche erhalten wurde, an einem wie oben beschriebenen Co/Cu/Mn/Mo-Katalysator gewonnen. Der vorhydrierte Einsatzstoff hatte einen Wassergehalt von ca. 52,5 Gew.-%. Als organische Komponenten enthielt er gemäß gaschromatografischer Analyse, wasserfrei gerechnet, 61,1 Gew.-% 1,6-Hexandiol sowie weitere Reaktionsprodukte des oben genannten Nebenproduktstroms. Die Säurezahl des vorhydrierten Einsatzstoffs betrug 6,5 mg KOH/g und war überwiegend auf 6-Hydroxycapronsäure sowie Spuren von Adipinsäure zurückzuführen. Weitere Komponenten im vorhydrierten Einsatzstoffs waren unter anderem 1-Hexanol, 1-Pentanol, 1,2-Cyclohexandiol, 1,3-Cyclohexandiol, 1,4-Cyclohexandiol, 1,5-Pentandiol, 1-Pentanol und 1,4-Butandiol.

Zur Hydrierung der 6-Hydroxycapronsäure sowie der Adipinsäure wurde der vorhydrierte Einsatzstoff kontinuierlich bei 230°C Reaktoreintrittstemperatur, 25 MPa abs und 50 Normliter/h Wasserstoff pro kg vorhydriertem Einsatzstoff in Rieselfahrweise über den Katalysator geleitet. Bei der vorliegenden Versuchsreihe wurde die Zulaufmenge an vorhydriertem Einsatzstoff sukzessive gesteigert, wobei die Probennahme jeweils ca 24 Stunden nach Einstellung einer Zulaufmenge erfolgte.

Die Ergebnisse sind in Tabelle 2 dargestellt. Der Wassergehalt wurde nach Karl-Fischer und der Gehalt an 1,4-Butandiol gaschromatografisch ermittelt.

Bei einer geringen Strömungsgeschwindigkeit von nur 5 m/h (Beispiel 5) werden die vorhandenen Carbonsäuren zwar weitgehend Vollständig hydriert, was sich in einer sehr geringen Säurezahl von < 0,5 mg KOH/g zeigt, jedoch werden dabei auch nennenswerte Mengen des Zielalkohols 1,6-Hexandiol durch Hydrierung abgebaut. So sinkt der Gehalt von 1,6-Hexandiol von ursprünglich 61,1 Gew.-% auf 60,2 Gew.-%, jeweils wasserfrei gerechnet.

Eine hohe Strömungsgeschwindigkeit von 60 m/h (Beispiel 8) ergibt zwar eine hohe rechnerische Durchsatzzahl durch den Reaktor, ermöglicht aber nur noch eine teilweise Hydrierung der vorhandenen Carbonsäuren. So weist der Austrag noch eine Säurezahl von 2,3 mg KOH/g auf, was einer Hydrierung von nur etwa 65% der vorhandenen Carbonsäuren entspricht.

Demgegenüber zeigen die Beispiele 6 und 7 mit einer Strömungsgeschwindigkeit von 10 beziehungsweise 30 m/h sowohl durch eine Säurezahl im Austrag von < 0,5 mg KOH/g eine nahezu vollständige Hydrierung der vorhandenen Carbonsäuren, als auch durch einen deutlich gestiegenen Gehalt an 1,6-Hexandiol von 64,0 beziehungsweise 64,1 Gew.-% eine deutliche Zunahme an Zielalkohol 1,6-Hexandiol.

Durch die erfindungsgemäße Hydrierung wurde aus einem Alkohol und Carbonsäure enthaltenden Einsatzstrom ein nahezu carbonsäurefreier Produktstrom mit einem deutlichen Zuwachs an Zielalkohol erhalten.

Beispiel 9

In Beispiel 9 (Vergleichsbeispiel) wurde das Langzeitverhalten bei der Hydrierung von 6-Hydroxycapronsäure zu 1,6-Hexandiol bei einer, auf die geometrische Querschnittsfläche des leeren, katalysatorfreien Reaktorrohres berechnete Strömungsgeschwindigkeit der durchströmenden Flüssigkeit von 60 m/h untersucht. Hierzu wurde zunächst Beispiel 8 wiederholt und über einen Zeitraum von 1000 Stunden unter diesen Bedingungen belassen. Dabei stiegt die Säurezahl im Austrag langsam von 2,3 auf 4,5 mg KOH/g. Entsprechend sank der Carbonsäure-Umsatz von rund 65 auf rund 30%. Gleichzeitig fanden sich geringe Mengen an Co und Mn von in Summe bis zu 10 Gew.-ppm im Austrag.

Das Vergleichsbeispiel 9 zeigt eine enorme Verschlechterung der Performance innerhalb von nur 1000 Stunden.

Beispiel 10

In Beispiel 10 wurde das Langzeitverhalten bei der Hydrierung von 6-Hydroxycapronsäure zu 1,6-Hexandiol bei einer, auf die geometrische Querschnittsfläche des leeren, katalysatorfreien Reaktorrohres berechnete Strömungsgeschwindigkeit der durchströmenden Flüssigkeit von 30 m/h untersucht. Hierzu wurde zunächst Beispiel 7 wiederholt und über einen Zeitraum von 3000 Stunden unter diesen Bedingungen belassen. Dabei stiegt die Säurezahl im Austrag langsam von < 0,5 auf gerade mal 0,6 mg KOH/g. Der Carbonsäure-Umsatz nach 3000 Stunden betrug somit immer noch über 90%. Co und Mn wurden nur in einer sehr geringer Menge von in Summe < 4 Gew.-ppm nachgewiesen.

Beispiel 10 zeigt bei einer Strömungsgeschwindigkeit von 30 m/h selbst nach 3000 Stunden Laufzeit nur einen geringfügigen Anstieg der Säurezahl beziehungsweise des Co und Mn-Gehalts im Austrag.

Tabelle 1

| Beispiel | Einsatzstoff [L/h] | Querschnittsbelastung[#1] [m/h] | Einsatzstoff | | | Austrag | | |
|---|---|---|---|---|---|---|---|---|
| | | | Säurezahl [mg KOH/g] | 1,4-BDO [#2] [Gew.-%] | Wasser [Gew.-%] | Säurezahl [mg KOH/g] | 1,4-BDO [#2] [Gew.-%] | Wasser [Gew.-%] |
| 1 (Vergleich) | 1,6 | 5 | 3,8 | 90,7 | 75,0 | < 0,5 | 88,0 | 75,5 |
| 2 (Erfindung) | 3,1 | 10 | | | | < 0,5 | 94,5 | 75,2 |
| 3 (Erfindung) | 9,4 | 30 | | | | < 0,5 | 94,3 | 75,1 |
| 4 (Vergleich) | 19 | 60 | | | | 1,5 | 93,0 | 75,1 |

[#1] Auf die geometrische Querschnittsfläche des leeren, katalysatorfreien Reaktorrohres berechnete Strömungsgeschwindigkeit der durchströmenden Flüssigkeit.
[#2] 1,4-Butandiol, wasserfrei gerechnet.

Tabelle 2

| Beispiel | Einsatzstoff [L/h] | Querschnittsbelastung[#1] [m/h] | Einsatzstoff | | | Austrag | | |
|---|---|---|---|---|---|---|---|---|
| | | | Säurezahl [mg KOH/g] | 1,6-HDO [#2] [Gew.-%] | Wasser [Gew.-%] | Säurezahl [mg KOH/g] | 1,6-HDO [#2] [Gew.-%] | Wasser [Gew.-%] |
| 5 (Vergleich) | 1,6 | 5 | 6,5 | 61,1 | 52,5 | < 0,5 | 60,2 | 53,5 |
| 6 (Erfindung) | 3,1 | 10 | | | | < 0,5 | 64,0 | 53,0 |
| 7 (Erfindung) | 9,4 | 30 | | | | < 0,5 | 64,1 | 52,9 |
| 8 (Vergleich) | 19 | 60 | | | | 2,3 | 61,0 | 52,7 |

[#1] Auf die geometrische Querschnittsfläche des leeren, katalysatorfreien Reaktorrohres berechnete Strömungsgeschwindigkeit der durchströmenden Flüssigkeit.
[#2] 1,6-Hexandiol, wasserfrei gerechnet.

**Patentansprüche**

1. Verfahren zur kontinuierlichen Hydrierung einer Carbonsäure der allgemeinen Formel (I)

$$Y^1\text{-}X^1\text{-}COOH \qquad (I)$$

in der $X^1$ für eine $\text{-}(CH_2)_n\text{-}$ Gruppe mit n gleich 1 bis 10 oder eine $\text{-}CH=CH\text{-}$Gruppe, und $Y^1$ für H-, HOOC- oder HO-$CH_2$- steht, oder eines Gemisches davon, unter Beibehaltung der Anzahl der Kohlenstoffatome zu einem Alkohol der allgemeinen Formel (II)

$$Y^2\text{-}X^2\text{-}CH_2OH \qquad (II)$$

in der $X^2$ für eine $\text{-}(CH_2)_n\text{-}$ Gruppe mit n gleich 1 bis 10, und $Y^2$ für H- oder HO-$CH_2$-steht, mit Wasserstoff bei einer Temperatur von 100 bis 300°C und einem Druck von 10 bis 33 MPa abs in einem axial durchströmten Reaktorrohr mit darin fixiertem Festbettkatalysator, welcher mindestens ein Element aus der Gruppe Re, Co und Cu enthält, **dadurch gekennzeichnet, dass** die zu hydrierende Carbonsäure (I) in einem flüssigen Gemisch (III) enthaltend die Carbonsäure (I), Wasser und Alkohol (II) vorliegt, wobei das Gemisch (III)

   a) eine Säurezahl von 0,2 bis 25 mg KOH/g aufweist,
   b) mindestens 15 Gew.-% Wasser,
   c) mindestens 20 Gew.-% Alkohol (II) enthält, und
   d) die, auf die geometrische Querschnittsfläche des leeren, katalysatorfreien Reaktorrohres berechnete Strömungsgeschwindigkeit der durchströmenden Flüssigkeit 10 bis 50 m/h beträgt.

2. Verfahren nach Anspruch 1, **dadurch gekennzeichnet, dass** das Gemisch (III) eine Säurezahl von 0,5 bis 10 mg KOH/g aufweist.

3. Verfahren nach den Ansprüchen 1 bis 2, **dadurch gekennzeichnet, dass** die Summe der Gehalte an Carbonsäure (I), Wasser und Alkohol (II) im Gemisch (III) $\leq$ 100 Gew.-% beträgt.

4. Verfahren nach Anspruch 3, **dadurch gekennzeichnet, dass** die Summe der Gehalte an Carbonsäure (I), Wasser und Alkohol (II) im Gemisch (III) 40 bis 100 Gew.-% beträgt.

5. Verfahren nach den Ansprüchen 1 bis 4, **dadurch gekennzeichnet, dass** die, auf die geometrische Querschnittsfläche des leeren, katalysatorfreien Reaktorrohres berechnete Strömungsgeschwindigkeit der durchströmenden Flüssigkeit 20-40 m/h beträgt.

6. Verfahren nach den Ansprüchen 1 bis 5, **dadurch gekennzeichnet, dass** man das Verfahren ohne Rückführung von hydriertem Gemisch durchführt.

7. Verfahren nach den Ansprüchen 1 bis 6, **dadurch gekennzeichnet, dass** das Gemisch (III) 10 bis 1000 Gew.-ppm Alkalimetall aus der Gruppe Na und K enthält.

8. Verfahren nach den Ansprüchen 1 bis 7, **dadurch gekennzeichnet, dass** man als Carbonsäure (I) Bernsteinsäure, 4-Hydroxybuttersäure, Maleinsäure, Glutarsäure, 5-Hydroxyvaleriansäure, Adipinsäure, 6-Hydroxycapronsäure oder ein Gemisch davon einsetzt.

9. Verfahren nach den Ansprüchen 1 bis 8, **dadurch gekennzeichnet, dass** man als Festbettkatalysator

   - einen Trägerkatalysator enthaltend 0,1 bis 10 Gew.-% Re auf einen Träger aus der Gruppe enthaltend Graphit, Aktivkohle, $ZrO_2$, $Al_2O_3$, $SiO_2$ und $TiO_2$,
   - einen Fällkatalysator enthaltend 1 bis 90 Gew.-% Co,
   - einen Fällkatalysator enthaltend 0,5 bis 60 Gew.-% Cu, oder
   - einen Fällkatalysator enthaltend 15 bis 85 Gew.-% Co und 5 bis 20 Gew.-% Cu,

   wobei die Summe aus dem Gehalt an Co und Cu 100 Gew.-% nicht übersteigt, einsetzt.

10. Verfahren nach den Ansprüchen 1 bis 9, **dadurch gekennzeichnet, dass** man das Gemisch (III) durch kontinuierliche Vorhydrierung einer Lösung enthaltend Carbonsäure (I) und Wasser gewinnt, wobei die Lösung eine Säurezahl von 50 bis 900 mg KOH/g aufweist.

**11.** Verfahren nach Anspruch 10, **dadurch gekennzeichnet, dass** man die Vorhydrierung mit Rückführung durchführt und 50 bis 98 Gew.-% der vorhydrierten Lösung zur Vorhydrierung rückführt.

**Claims**

**1.** A process for the continuous hydrogenation of a carboxylic acid of the general formula (I)

$$Y^1\text{-}X^1\text{-}COOH \qquad (I)$$

where $X^1$ is a $-(CH_2)_n-$ group with n from 1 to 10 or a -CH=CH- group and $Y^1$ is H-, HOOC- or HO-$CH_2$-, or a mixture thereof, with retention of the number of carbon atoms to give an alcohol of the general formula (II)

$$Y^2\text{-}X^2\text{-}CH_2OH \qquad (II)$$

where $X^2$ is a $-(CH_2)_n-$ group with n from 1 to 10 and $Y^2$ is H- or HO-$CH_2$-, by means of hydrogen at a temperature of from 100 to 300°C and a pressure of from 10 to 33 MPa abs in a reactor tube through which axial flow occurs and which has a fixed-bed catalyst which is fixed therin and comprises at least one element from the group consisting of Re, Co and Cu, wherein the carboxylic acid (I) to be hydrogenated is present in a liquid mixture (III) comprising the carboxylic acid (I), water and alcohol (II), where the mixture (III)

a) has an acid number of from 0.2 to 25 mg KOH/g,
b) comprises at least 15% by weight of water,
c) comprises at least 20% by weight of alcohol (II) and
d) the flow velocity of the flowing liquid calculated on the basis of the geometric cross-sectional area of the empty, catalyst-free reactor tube is from 10 to 50 m/h.

**2.** The process according to claim 1, wherein the mixture (III) has an acid number of from 0.5 to 10 mg KOH/g.

**3.** The process according to either of claims 1 and 2, wherein the sum of the contents of carboxylic acid (I), water and alcohol (II) in the mixture (III) is $\leq$ 100% by weight.

**4.** The process according to claim 3, wherein the sum of the contents of carboxylic acid (I), water and alcohol (II) in the mixture (III) is from 40 to 100% by weight.

**5.** The process according to any of claims 1 to 4, wherein the flow velocity of the flowing liquid calculated on the basis of the geometric cross-sectional area of the empty, catalyst-free reactor tube is 20-40 m/h.

**6.** The process according to any of claims 1 to 5, wherein the process is carried out without recirculation of hydrogenated mixture.

**7.** The process according to any of claims 1 to 6, wherein the mixture (III) comprises from 10 to 1000 ppm by weight of alkali metal from the group consisting of Na and K.

**8.** The process according to any of claims 1 to 7, wherein succinic acid, 4-hydroxybutyric acid, maleic acid, glutaric acid, 5-hydroxyvaleric acid, adipic acid, 6-hydroxycaproic acid or a mixture thereof is used as carboxylic acid (I).

**9.** The process according to any of claims 1 to 8, wherein

- a supported catalyst comprising from 0.1 to 10% by weight of Re on a support from the group comprising graphite, activated carbon, $ZrO_2$, $Al_2O_3$, $SiO_2$ and $TiO_2$,
- a precipitated catalyst comprising from 1 to 90% by weight of Co,
- a precipitated catalyst comprising from 0.5 to 60% by weight of Cu or
- a precipitated catalyst comprising from 15 to 85% by weight of Co and from 5 to 20% by weight of Cu,

where the sum of the contents of Co and Cu does not exceed 100% by weight,
is used as fixed-bed catalyst.

10. The process according to any of claims 1 to 9, wherein the mixture (III) is obtained by continuous prehydrogenation of a solution comprising carboxylic acid (I) and water, with the solution having an acid number of from 50 to 900 mg KOH/g.

11. The process according to claim 10, wherein the prehydrogenation is carried out with recirculation and from 50 to 98% by weight of the prehydrogenated solution is recirculated to the prehydrogenation.


**Revendications**

1. Procédé d'hydrogénation continue d'un acide carboxylique de la formule générale (I) :

$$Y^1\text{-}X^1\text{-}COOH \qquad (I)$$

dans laquelle $X^1$ représente un groupe $-(CH_2)_n-$ avec n représentant 1 à 10, ou un groupe -CH=CH-, et $Y^1$ représente H-, HOOC- ou HO-$CH_2$-, ou un mélange de ceux-ci, avec conservation du nombre d'atomes de carbone pour former un alcool de la formule générale (II) :

$$Y^2\text{-}X^2\text{-}CH_2OH \qquad (II)$$

dans laquelle $X^2$ représente un groupe $-(CH_2)_n-$ avec n représentant 1 à 10, et $Y^2$ représente H- ou HO-$CH_2$-, avec de l'hydrogène à une température de 100 à 300 °C et à une pression de 10 à 33 MPa abs dans un tube de réacteur traversé axialement dans lequel est fixé un catalyseur en lit fixe, qui contient au moins un élément du groupe constitué par Re, Co et Cu, **caractérisé en ce que** l'acide carboxylique (I) à hydrogéner se présente dans un mélange liquide (III) contenant l'acide carboxylique (I), de l'eau et un alcool (II), le mélange (III)

  a) présentant un indice d'acidité de 0,2 à 25 mg de KOH/g,
  b) contenant au moins 15 % en poids d'eau,
  c) contenant au moins 20 % en poids d'alcool (II), et
  d) la vitesse d'écoulement du liquide traversant calculée sur la surface de section transversale géométrique du tube de réacteur vide sans catalyseur étant de 10 à 50 m/h.

2. Procédé selon la revendication 1, **caractérisé en ce que** le mélange (III) présente un indice d'acidité de 0,5 à 10 mg de KOH/g.

3. Procédé selon les revendications 1 et 2, **caractérisé en ce que** la somme des teneurs en acide carboxylique (I), eau et alcool (II) dans le mélange (III) est ≤ 100 % en poids.

4. Procédé selon la revendication 3, **caractérisé en ce que** la somme des teneurs en acide carboxylique (I), eau et alcool (II) dans le mélange (III) est de 40 à 100 % en poids.

5. Procédé selon les revendications 1 à 4, **caractérisé en ce que** la vitesse d'écoulement du liquide traversant calculée sur la surface de section transversale géométrique du tube de réacteur vide sans catalyseur est de 20 à 40 m/h.

6. Procédé selon les revendications 1 à 5, **caractérisé en ce que** le procédé est réalisé sans recyclage du mélange hydrogéné.

7. Procédé selon les revendications 1 à 6, **caractérisé en ce que** le mélange (III) contient 10 à 1 000 ppm en poids d'un métal alcalin du groupe constitué par Na et K.

8. Procédé selon les revendications 1 à 7, **caractérisé en ce que** l'acide succinique, l'acide 4-hydroxybutyrique, l'acide maléique, l'acide glutarique, l'acide 5-hydroxyvalérique, l'acide adipique, l'acide 6-hydroxycapronique ou un mélange de ceux-ci est utilisé en tant qu'acide carboxylique (I).

9. Procédé selon les revendications 1 à 8, **caractérisé en ce que**

- un catalyseur supporté contenant 0,1 à 10 % en poids de Re sur un support du groupe contenant le graphite, le charbon actif, $ZrO_2$, $Al_2O_3$, $SiO_2$ et $TiO_2$,
- un catalyseur précipité contenant 1 à 90 % en poids de Co,
- un catalyseur précipité contenant 0,5 à 60 % en poids de Cu, ou
- un catalyseur précipité contenant 15 à 85 % en poids de Co et 5 à 20 % en poids de Cu, la somme de la teneur en Co et Cu ne dépassant pas 100 % en poids,

est utilisé en tant que catalyseur en lit fixe.

10. Procédé selon les revendications 1 à 9, **caractérisé en ce que** le mélange (III) est obtenu par pré-hydrogénation continue d'une solution contenant l'acide carboxylique (I) et de l'eau, la solution présentant un indice d'acidité de 50 à 900 mg de KOH/g.

11. Procédé selon la revendication 10, **caractérisé en ce que** la pré-hydrogénation est réalisée avec recyclage, et 50 à 98 % en poids de la solution pré-hydrogénée est recyclée dans la pré-hydrogénation.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5698749 A
- US 3478112 A
- US 20110124926 A
- DE 2321101
- US 4940805 A
- US 20030114719 A
- DE 2321101 A